# EUROPÄISCHE PATENTSCHRIFT

(11) **EP 0 340 341 B1**
(45) Veröffentlichungstag und Bekanntmachung des Hinweises auf die Patenterteilung: **06.10.1993**
(21) Anmeldenummer: 88119791.7
(22) Anmeldetag: 28.11.1988
(51) Int. Cl.: C12M 3/00

(54) **Begasungs-Brutschrank**
Aerated incubator
Incubateur aéré

(30) Priorität: 06.05.1988 DE 3815528
(43) Veröffentlichungstag der Anmeldung: 08.11.1989
(73) Patentinhaber: Heraeus Instruments GmbH, 63450 Hanau (DE)
(72) Erfinder: Heeg, Hubert, D-6000 Frankfurt (DE); Fenner, Manfred, D-8752 Kleinkahl (DE); Werner, Hans-Peter, D-6073 Egelsbach (DE); Schmidt, Olaf, D-6078 Neu Isenburg (DE)
(74) Vertreter: Kühn, Hans-Christian

(56) Entgegenhaltungen:
- EP-A- 0 238 313
- DE-A- 2 259 377
- US-A- 4 039 775

## Beschreibung

Die vorliegende Erfindung betrifft einen Begasungs-Brutschrank zum Kultivieren von menschlichen und tierischen Zellen oder Geweben mit einem mit einer Außentür verschließbaren Innengehäuse, das von einem wärmeisolierenden äußeren Gehäuse umgeben ist, wobei das äußere Gehäuse mit Abstand zu den Seitenwänden, der Rückwand und der Decke angeordnet ist, wobei elektrische Heizkörper unterhalb des Bodens des Innengehäuses und im Bereich der Seitenwände angeordnet sind, und mit einer Befeuchtungsvorrichtung für die Innenraumatmosphäre.

Ein solcher Brutschrank ist beispielsweise aus der GB-A 231 259 bekannt. Zwischen dem Innenbehälter und dem Außenbehälter ist ein Rohrheizkörper angeordnet, der einteilig mäanderförmig die beiden Seitenwände und den Boden vollständig umläuft. Sowohl die Innenwände als auch der Boden des Innenbehälters weisen Öffnungen auf, durch die die in dem Zwischenraum durch den Rohrheizkörpers erwärmte Luft in den Nutzraum des Schrankes zirkulieren kann. Die Luftströmung zwischen dem Nutz- oder Innenraum und dem Zwischenraum, in dem der Rohrheizkörper angeordnet ist, wird durch einen Lüfter in der Rückwand erhalten, der die Luft aus dem Nutzraum in eine Kammer an der Rückseite des Brutschrankes ansaugt und über eine Bodenkammer dem Zwischenraum wieder zuführt. Über einen Umluftkanal kann der Nutzraum zusätzlich mit einer Kühleinheit verbunden werden, die ein zusätzliches Gebläse sowie einen Verdampfer aufweist.

Um die Luft im Nutzraum solcher Brutschränke zu befeuchten, werden zwei verschiedene Arten von Luftbefeuchtungseinrichtungen eingesetzt. Nach der einen Art wird Wasser aus einem Wasservorrat außerhalb des Nutzraumes verdampft und das Aerosol dem Nutzraum zugeführt. Eine andere Art, die Nutzraumluft zu befeuchten, besteht darin, einen Wasservorrat direkt im Nutzraum anzuordnen und dort zu verdampfen. Üblicherweise wird hierzu eine Wasserwanne auf dem Boden des Nutzraumes angeordnet und mit einer geeigneten Heizvorrichtung, die direkt in dem Wasserbad angeordnet sein kann, beheizt.

Es ist festzustellen, daß die bekannten Brutschränke hinsichtlich einer gleichmäßigen Temperaturverteilung entlang der Prüfrauminnenwände sehr unterschiedliche Ergebnisse erzielen. So neigen Brutschränke, deren Innenbehälter eine ungleichmäßige Temperaturverteilung aufweisen, zu Kondensatbildungen an den kälteren Bereichen der Wände, so daß hohe Luftfeuchtigkeiten, die im Bereich von 90 % und mehr liegt, nur schwer zu erreichen sind.

Ausgehend von dem vorstehend aufgezeigten Stand der Technik liegt der vorliegenden Erfindung die Aufgabe zugrunde, einen Brutschrank der eingangs beschriebenen Art derart auszubilden, daß eine hohe relative Feuchte im Prüfraum erreichbar ist, bei dem eine schnelle und gleichmäßige Aufheizung des Wassers ermöglicht wird, bei dem Kondensatbildung an der Behälterinnenwand weitgehendst vermieden wird und dessen Beheizungssystem kostengünstig herstellbar ist.

Die Aufgabe wird dadurch gelöst, daß die elektrischen Heizelemente Rohrheizkörper sind, die direkt an der Außenseite des Bodens des Innengehäuses, der eine Befeuchtungsvorrichtung in Form einer ein Wasserbad aufnehmenden Wanne aufweist, flächig anliegen, und im Bereich der Seitenwände und der Rückwand des Innengehäuses so nach oben angewinkelt sind, daß sie die Bodenebene des Innengehäuses etwas über die Höhe des Wasserbades überragen und so den Übergangsbereich zwischen Boden und Seiten- bzw. Rückwand beheizen, wobei die den Seitenwänden und der Rückwand zugewandten Rohrheizkörper von diesen Wänden beabstandet sind.

Durch die Anordnung der Rohrheizkörper am Boden sowie im unteren Bereich der Seitenwände und der Rückwand wird gezielt der Wasservorrat beheizt. Gleichzeitig wird aber der Zwischenraum zwischen dem Innengehäuse und dem wärmeisolierten äußeren Gehäuse gleichmäßig durch die aufsteigende Warmluft aufgeheizt. Die Erwärmung der Innengehäusewände durch die Konvektion im Behälterzwischenraum führt zu einer gleichmäßigen Temperaturverteilung im Nutzraum, so daß eine Kondensatbildung an den Innenwänden des Innengehäuses nicht auftritt. Weiterhin werden durch die besondere Anordnung des oder der Rohrheizkörper insbesondere die Eckbereiche des Innengehäuses, d. h. die Übergangsbereiche zwischen dem Boden und den Seitenwänden bzw. der Rückwand, aufgeheizt, wobei von Vorteil die Rohrheizkörper an der Außenseite des Bodens des Innengehäuses direkt anliegen, so daß eine direkte Beheizung des Wassers das mittels einer Wanne, die auf dem Boden des Innengehäuses aufsteht, bevorratet ist, erfolgt. Im Gegensatz zu dem im Bereich des Behälterbodens angeordneten Rohrheizkörper sind die Rohrheizkörper im Bereich der Seitenwände und der Rückwand freistrahlend angeordnet, d. h. sie liegen nicht an den Behälterwänden an, so daß keine Bereiche erhöhter Temperatur auftreten. Diese Maßnahme ist insbesondere in dem Bereich der Seitenwand und der Rückwand wesentlich, die oberhalb des Spiegels des Wasserbades liegt. Durch die, wenn überhaupt, geringe Kondensatbildung an den Wänden des Innengehäuses können relative Feuchten erreicht und eingestellt werden, die oberhalb von 95 % liegen.

Vorzugsweise sollte eine zur Bevorratung des Wassers eingesetzte Wanne auf dem Boden flächig anliegen, um einen direkten Wärmeübergang zur Beheizung des Wassers zu erzielen. Dieser direkte Wärmeübergang kann dadurch verstärkt werden, daß der Boden des Innengehäuses direkt als Wanne, beispielsweise in Form eines tiefgezogenen Edelstahlteiles, ausgebildet ist. Je nach Größe des erforderlichen Wasservorrates kann der gesamte Boden des Innenraumes eine Wanne bilden, wodurch gleichzeitig eine große Verdunstungsoberfläche erhalten wird.

Die eingesetzten Rohrheizkörper sind vorzugsweise mäanderförmig verlaufend gebogen, so daß sie insbesondere die hinteren unteren Eckbereiche des Innengehäuses gleichmäßig umschließen und beheizen. In einer bevorzugten Auführungsform werden zwei Rohrheizkörper eingesetzt, wobei jeder dieser Rohrheizkörper einen der erwähnten Eckbereiche umschließt, indem mehrere mäanderförmige Windungen des jeweiligen Rohrheizkörpers in drei senkrecht zueinander stehenden Ebenen verlaufen. Um Kondensat auf der Glastür zu vermeiden, kann ein Heizkörper in die Tür (Folienheizung oder Widerstandsheizspirale) zum Beheizen der Tür eingebaut werden.

Weitere Einzelheiten und Merkmale der Erfindung ergeben sich aus der nachfolgenden Beschreibung eines Ausführungsbeispiels anhand der Zeichnung. In der Zeichnung zeigt
- Figur 1: eine perspektivische Ansicht eines Begasungs-Brutschrankes,
- Figur 2: ein Schnittbild eines Begasungs-Brutschrankes,

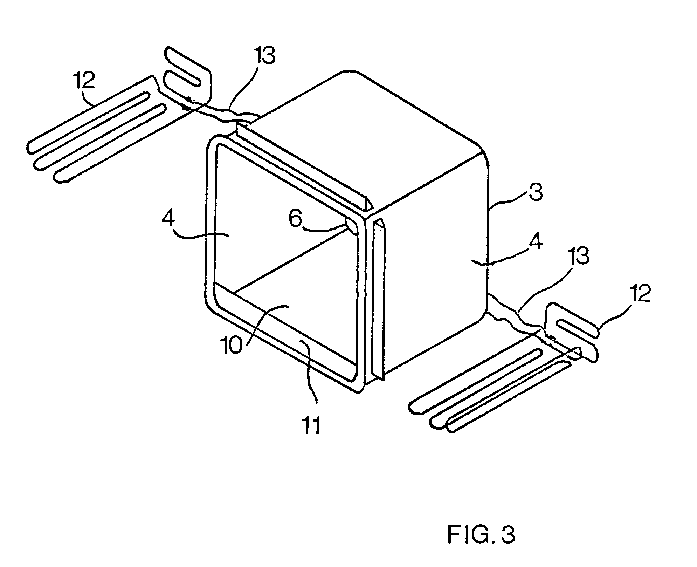

- Figure 3: einen Innenbehäler, wie er im Brutschrank nach Figur 1 eingesetzt ist, mit zwei dem Innengehäuse zugeordneten Rohrheizkörpern.

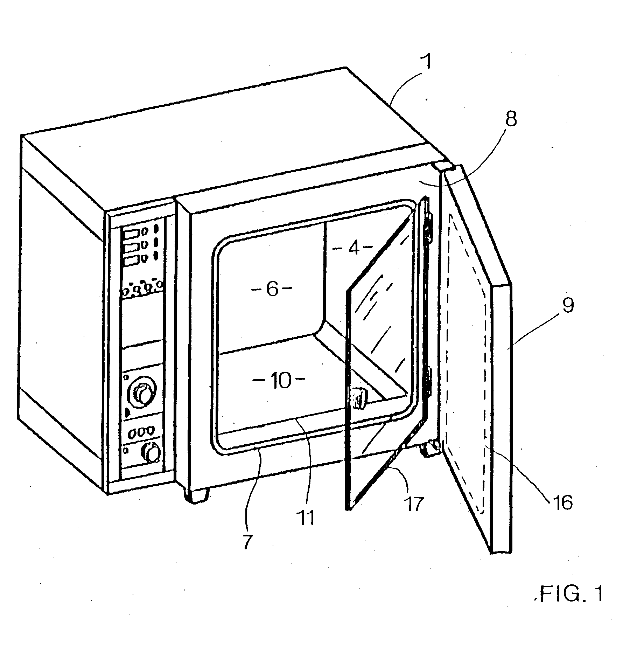

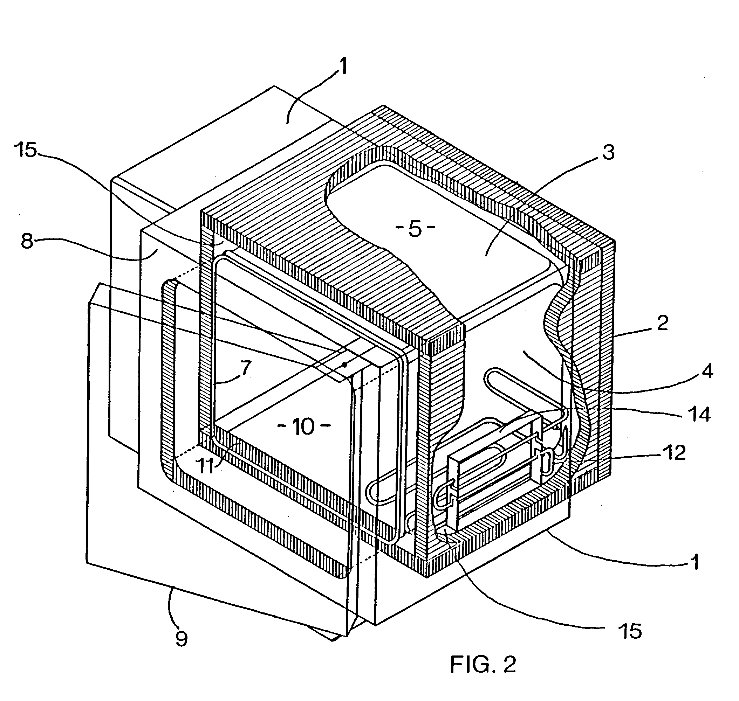

Der Brutschrank, wie er in den Figuren 1 und 2 gezeigt ist, weist ein äußeres Gehäuse 1 auf, das mit einem freitragenden Wärmeisolationskörper 2 auf seiner Innenseite ausgekleidet ist. In das äußere Gehäuse 1 ist mit Abstand zu diesem bzw. zu der Innenseite des Wärmeisolationskörpers 2 ein Innengehäuse eingesetzt, so daß zwischen dem Innengehäuse 3 und dem Wärmeisolationskörper 2 ein Zwischenraum, zumindest im Bereich der Seitenwände 4 und der Oberseite 5 entsteht. Die Rückwand des Brutschrankes, die in dieser Frontansicht nicht zu sehen ist, ist ebenfalls wärmeisoliert und mit Abstand zur Rückwand 6 des Innengehäuses 3 angeordnet. In der Darstellung nach Figur 2 ist sowohl der Wärmeisolationskörper 2 als auch das Innengehäuse 3 geringfügig nach hinten aus dem äußeren Gehäuse 1 herausgezogen. Das Innengehäuse weist an seiner Vorderseite eine um den Rand umlaufende Dichtlippe 7 auf, die durch den Frontrahmen 8 hindurchragt und gegen die sich eine Tür 9, beispielsweise eine Glastür, dichtend anlegt. Der Boden des Innengehäuses 3 ist als Wanne 10 ausgebildet, wozu im Bereich der Öffnung des Innengehäuses ein Wannenrand 11 eingesetzt ist. Diese Wanne 10 kann annähernd bis zur Oberkante ihres Wannenrandes 11 mit Wasser angefüllt werden.

Zum Beheizen des Innenraumes des Innengehäuses 3 sind zwei Rohrheizkörper 12 vorgesehen, die den Boden des Innengehäuses 3 sowie die Seitenwände 4 als auch die Rückwand 6 im unteren Bereich, in dem auch die Wanne 10 gebildet ist, umschließen. Jeder der beiden Rohrheizkörper 12 ist einteilig aus einem Rohr mäanderförmig geboden, wobei zwei Mäander dem Boden, ein Mäander der Seitenwand 4 und zwei Mäander der Rückwand 6 zugeordnet sind. Die drei Ebenen, die durch diese Mäander gebildet sind, stehen senkrecht zueinander. Die beiden Anschlußenden 13 jedes Rohrheizkörpers 12 sind in den Bereich der Mitte der Rückwand 6 gelegt, so daß sie gut zugänglich sind. Die Windungen des Rohrheizkörpers 12 im Bereich der Seitenwand 4 und der Rückwand 6 enden so, daß die Wanne 10 ausreichend beheizt wird. Insbesondere die Kanten und der Eckbereich des Innengehäuses 3 werden durch die beiden Rohrheizkörper 12 beheizt. Die Rohrheizkörper 12, die am Boden des Innengehäuses 3, der gleichzeitig den Boden der Wanne 10 für den Wasservorrat bildet, anliegen, sind von den Seitenwänden 4 und der Rückwand 6 beabstandet sind. Im Bereich der Seitenwände 4, gegebenenfalls im Bereich der Rückwand, werden die Rohrheizkörper durch einen Trägerrahmen 14, beispielsweise aus Blech, gehalten, wie dies aus Figur 1 ersichtlich ist. Durch die Rohrheizkörper 12 wird einerseits das in der Wanne 10 bevorratete Wasser aufgeheizt, so daß es verdampft; gleichzeitig wird der zwischen dem Innengehäuse 3 und dem Wärmeisolationskörper 2 gebildete Zwischenraum 15 durch Wärmekonvektion gleichmäßig aufgeheizt.

Der Wannenrand 11 ist in einer Höhe von etwa 40 mm ausgeführt, so daß das Wasser in einer Höhe von 15 bis 20 mm angefüllt werden kann. Für einen Nutzraum mit einem Volumen von etwa 83 l mit kubischen Maßen sollte die Oberfläche des Wasserspiegels etwa 0,2 m², das Volumen des Zwischenraumes 15 im Bereich der Seitenwände 4 etwa 9 l, im Bereich der Decke etwa 5 l und im Bereich der Rückwand 6 etwa 7 l betragen, woraus sich ein Gesamtvolumen des Konvektions- oder Zwischenraumes 15 von etwa 21 l ergibt. Für einen Brutschrank mit einem Volumen des Nutzraumes von etwa 250 l sollte die Wasseroberfläche etwa 0,4 m², der Wasservorrat etwa 5 bis 6 l, das Volumen des Zwischenraumes 15, im Bereich der Seitenwände 4 etwa 21 l, im Bereich der Decke etwa 9 l und im Bereich der Rückwand 6 etwa 15 l betragen. In dieser Dimensionierung wird bei entsprechend ausgelegtem Rohrheizkörper 12 der sich über eine Höhe von etwa 10 cm im Bereich der Seitenwände 4 und der Rückwand 6 erstrecken soll, eine optimale Wasserverdunstung und Aufheizung der Wände des Innengehäuses 3 erreicht.

## Patentansprüche

1. Begasungs-Brutschrank zum Kultivieren von menschlichen oder tierischen Zellen oder Geweben mit einem mit einer Tür verschließbaren Innengehäuse, das von einem wärmeisolierenden äußeren Gehäuse umgeben ist, wobei das äußere Gehäuse mit Abstand zu den Seitenwänden, der Rückwand und der Decke angeordnet ist, wobei elektrische Heizkörper unterhalb des Bodens des Innengehäuses und im Bereich der Seitenwände angeordnet sind, und mit einer Befeuchtungsvorrichtung für die Innenraumatmosphäre, dadurch gekennzeichnet, daß die elektrischen Heizelemente Rohrheizkörper (12) sind, die direkt an der Außenseite des Bodens des Innengehäuses (3), der eine Befeuchtungsvorrichtung in Form einer ein Wasserbad aufnehmenden Wanne (10) aufweist, flächig anliegen, und im Bereich der Seitenwände (4) und der Rückwand (6) des Innengehäuses (3) so nach oben angewinkelt sind, daß sie die Bodenebene des Innengehäuses (3) die Höhe des Wasserbades überragen und so den Übergangsbereich zwischen Boden und Seiten- (4) bzw. Rückwand (6) beheizen, wobei die den Seitenwänden (4) und der Rückwand (6) zugewandten Rohrheizkörper (12) von diesen Wänden (4,6) beabstandet sind.

2. Begasungs-Brutschrank nach Anspruch 1, dadurch gekennzeichnet, daß die Wanne (10) an der Rückwand (6) und mindestens im hinteren, an die Rückwand (6) angrenzenden Bereich der Seitenwand (4) anliegt.

3. Begasungs-Brutschrank nach Anspruch 1 oder 2, dadurch gekennzeichnet, daß der Boden des Innengehäuses (3) als Wanne (10) ausgebildet ist.

4. Begasungs-Brutschrank nach einem der Ansprüche 1 bis 3, dadurch gekennzeichnet, daß der/die Rohrheizkörper (12) mäanderförmig gebogen ist/sind.

5. Begasungs-Brutschrank nach Anspruch 4, dadurch gekennzeichnet, daß jedem hinteren, unteren Eckbereich des Innengehäuses (3) ein einteiliger Rohrheizkörper (12) zugeordnet ist, dessen mäanderförmige Windungen in drei senkrecht aufeinanderstehenden Ebenen verlaufen.

6. Begasungs-Brutschrank nach einem der Ansprüche 1 bis 5, dadurch gekennzeichnet, daß in der Tür (9) ein Heizkörper (16) angeordnet ist.

## Claims

1. A gas incubator for the cultivation of human or animal cells or tissues with an inner housing, which is able to be closed by a door and which is surrounded by a thermally insulating outer housing, in which the outer housing is arranged at a distance from the side walls, the rear wall and the lid, in which electric heating bodies are arranged beneath the base of the inner housing and in the region of the side walls, and with a humidifying device for the interior atmosphere, characterised in that the electric heating elements are tubular heating bodies (12), which lie flat directly against the outer side of the base of the inner housing (3), which has a humidifying device in the form of a trough (10) holding a water bath, and which heating bodies (12) in the region of the side walls (4) and of the rear wall (6) of the inner housing (3) are angled upwards such that they project over the base plane of the inner housing (3) by the height of the water bath and thus heat the transition region between base and side wall (4) or rear wall (6), in which the tubular heating bodies (12), facing the side walls (4) and the rear wall (6) are distanced from these walls (4, 6).

2. A gas incubator according to Claim 1, characterised in that the trough (10) lies against the rear wall (6) and at least in the rear region of the side wall (4) adjoining the rear wall (6).

3. A gas incubator according to Claim 1 or 2, characterised in that the base of the inner housing (3) is constructed as a trough (10).

4. A gas incubator according to one of Claims 1 to 3, characterised in that the tubular heating body/bodies (12) is/are bent in a meander shape.

5. A gas incubator according to Claim 4, characterised in that there is associated with each rear, lower corner region of the inner housing (3) a one-piece tubular heating body (12) the meander-shaped windings of which run in three planes standing perpendicular to each other.

6. A gas incubator according to one of Claims 1 to 5, characterised in that a heating body (16) is arranged in the door (9).

## Revendications

1. Armoire d'incubation sous atmosphère, pour la culture de cellules ou tissus humains ou animaux, comportant une enceinte intérieure pouvant être fermée par une porte et entourée par une enceinte extérieure thermiquement isolée, l'enceinte extérieure étant disposée à distance des parois latérales, de la paroi arrière et de la paroi supérieure, des éléments chauffants électriques étant agencés en dessous du fond ou plancher de l'enceinte intérieure et dans la région des parois latérales; et un dispositif d'humidification de l'atmosphère de l'espace intérieur, caractérisée par le fait que les éléments chauffants électriques sont des éléments chauffants tubulaires (12) qui sont appliqués en surface directement contre le côté extérieur du fond de l'enceinte intérieure (3), laquelle présente un dispositif d'humidification sous la forme d'une cuve (10) recevant un bain d'eau, ces éléments chauffants tubulaires étant coudés vers le haut, dans la région des parois latérales (4) et de la paroi arrière (6) de l'enceinte intérieure (3), de manière telle qu'ils dépassent, au-delà du plan du fond de l'enceinte intérieure (3), la hauteur du bain d'eau et chauffent ainsi la zone de transition entre fond et paroi latérale (4) et/ou paroi arrière (6), les éléments chauffants tubulaires (12) tournés vers les parois latérales (4) et la paroi arrière (6) étant écartés de ces parois (4, 6).

2. Armoire d'incubation selon revendication 1, caractérisée par le fait que la cuve (10) s'applique contre la paroi arrière (6) et au moins dans la région arrière de la paroi latérale (4) bordant la paroi arrière (6).

3. Armoire d'incubation selon revendication 1 ou 2, caractérisée par le fait que le fond de l'enceinte intérieure (3) est réalisé en tant que cuve (10).

4. Armoire d'incubation selon l'une des revendications 1 à 3, caractérisée par le fait que le ou les éléments chauffants tubulaires (12) est ou sont coudé(s) en forme de méandres.

5. Armoire d'incubation selon revendication 4, caractérisée par le fait qu'à chaque région d'un coin arrière inférieur de l'enceinte intérieure (3) est associé un élément chauffant tubulaire (12) d'une seule pièce, dont les spires en méandres s'étendent dans trois plans orthogonaux.

6. Armoire d'incubation selon l'une des revendications 1 à 5, caractérisée par le fait qu'un élément chauffant (16) est agencé dans la porte (9).
